# EUROPEAN PATENT APPLICATION

(11) **EP 4 321 527 A1**
(43) Date of publication of application: **14.02.2024**
(21) Application number: 22784856.1
(22) Date of filing: 31.03.2022
(51) Int. Cl.: C07K 14/00, C07K 7/08, A61K 38/00, A61P 17/02, A61P 17/06

(54) **CELL-PENETRATING PEPTIDE VARIANT AND USE THEREOF**

(30) Priority: 07.04.2021 KR 20210045245
(71) Applicant: Regen Innopharm Inc., Seoul 06591 (KR)
(72) Inventor: OH, Il Hoan, Seoul 06084 (KR)
(74) Representative: Isarpatent
(86) International application number: PCT/KR2022/004581
(87) International publication number: WO 2022/215946

(57) **Abstract**

The present invention relates to use of cell-penetrating peptide variants for wound healing. Cell-penetrating peptides according to the present invention have the effect of improving cell permeation, cell proliferation, cell migration and blood vessel formation, and thus can be used for cell-penetrating delivery, wound healing, and angiogenesis promotion. Particularly, the cell-penetrating peptides have an excellent effect of treating diabetic wounds (ulcers), which are intractable, and thus can be effectively used for wound healing.

## Description

### [Technical Field]

The present invention relates to use of cell-penetrating peptide variants for wound healing.

### [Background Art]

The cells of higher animals have a plasma membrane that separates the inside and outside of the cell, and serves to thoroughly distinguish the movement of substances between cells. The cell membrane consists of a phospholipid bilayer, and due to the hydrophobicity of these lipid bilayers, it is almost impossible for most peptides, proteins, nucleotides, liposomes, etc. to move into cells. Accordingly, the cell membrane serves as an obstacle to move peptides, proteins, or compound drugs or gene therapeutic agents into the cell. To overcome this problem, methods of using cationic lipids or polyethyleneimine (PEI), viral vectors, or electroporation have been frequently used. However, these methods may have limitations due to low efficiency, limitations in applicable cells, and intracellular toxicity (Lindsay MA (2002) Peptide-mediated cell delivery: application in protein target validation. Curr Opin Pharmacol 2: 587-594, Green I, Christison R, Voyce CJ, Bundell KR, Lindsay MA (2003) Protein transduction domains: are they delivering Trends Pharmacol Sci 24: 213-215). In addition, as the functions of major proteins that play an important role in the occurrence and progression of diseases have been found, low-molecular compounds have been used as main drugs as substances capable of regulating disease-related proteins in cells, but it is known that large-area protein-protein interaction sites have limitations in controlling the activity of target proteins by binding to small molecule drugs. To overcome this problem, attempts have been made to develop peptide or protein-based drugs suitable for controlling protein-protein interactions. For the successful development of protein drugs for the purpose of controlling the activity of target proteins present in cells, intracellular delivery and transport of proteins are necessarily required, but the intracellular transport of proteins has generally been known to be difficult.

To overcome these disadvantages and limitations, attempts haven been made to use a protein transduction domain (PTD), that is, a cell-penetrating peptide (CPP). The CPP is also called a protein transduction domain (PTD). The CPP is part of a translocatory protein, and representative examples include membrane translocating sequence (MTS), which is a hydrophobic region in a signal sequence of human fibroblast growth factor 4, and Tat-PTD (basic amino acid domain) of Tat protein which is one of viral proteins of HIV (Mann DA, Frankel AD (1991) Endocytosis and targeting of exogenous HIV-1 Tat protein. EMBO J 10: 1733-1739, Rojas M, Donahue JP, Tan Z, Lin YZ (1998) Genetic engineering of proteins with cell membrane permeability. Nat Biotechnol 16: 370-375).

However, the MTS is recognized as a good candidate due to its very high efficiency of entering cells. However, since most of the amino acid sequence is a hydrophobic domain having hydrophobicity, there is a possibility that inclusion bodies may be generated during the expression process for production, there is a disadvantage of performing more complicated steps for expression and purification, and in spite of purified proteins, there is a disadvantage that the proteins are aggregated during storage due to hydrophobic interactions to deteriorated the activities thereof (Jo D, Nashabi A, Doxsee C, Lin Q, Unutmaz D, Chen J, Ruley HE (2001) Epigenetic regulation of gene structure and function with a cell-permeable Cre recombinase. Nat Biotechnol 19:929-933, DiGiandomenico A, Wylezinski LS, Hawiger J (2009) Intracellular delivery of a cell-penetrating SOCS1 that targets IFN-gamma signaling. Sci Signal 2: ra37). On the other hand, in the case of basic domains such as Tat-PTD, which mainly contain basic amino acids, there is a low possibility that insoluble substances may be formed during the expression process for production due to hydrophilicity, and there is an advantage of being purified from the cytoplasm by a relatively easy method and maintaining its activity for a relatively long period of time during storage. In addition, a lot of PTDs have been reported so far (Lindgren M, Langel U (2011) Classes and prediction of cell-penetrating peptides. Methods Mol Biol 683: 3-19).

Research to find several cell-penetrating peptides and reveal their sequences has been actively conducted around the world, and both Tat-PTD series and MTS series are used as cell-penetrating drugs for direct treatment purposes, but the effect thereof is minimal compared to other treatment methods such as gene therapy.

### [Disclosure]

### [Technical Problem]

An object of the present invention is to provide a cell-penetrating peptide.

Another object of the present invention is to provide a composition for promoting cell proliferation and migration.

Yet another object of the present invention is to provide a cell-penetrating carrier.

Yet another object of the present invention is to provide a composition for tissue regeneration.

Yet another object of the present invention is to provide a composition for promoting angiogenesis.

Yet another object of the present invention is to provide a pharmaceutical composition for wound healing.

Yet another object of the present invention is to provide a pharmaceutical composition for preventing or treating angiogenesis-dependent diseases.

Yet another object of the present invention is to provide a method for wound healing.

Yet another object of the present invention is to provide a method for treating angiogenesis-dependent diseases.

Yet another object of the present invention is to provide a use for preparing a composition for wound healing.

Yet another object of the present invention is to provide a use of for preparing a composition for preventing or treating angiogenesis-dependent diseases.

### [Technical Solution]

An aspect of the present invention provides a cell-penetrating peptide.

Another aspect of the present invention provides a composition for promoting cell proliferation and migration including a cell-penetrating peptide.

Yet another aspect of the present invention provides a cell-penetrating carrier including a cell-penetrating peptide.

Yet another aspect of the present invention provides a composition for tissue regeneration including a cell-penetrating peptide.

Yet another aspect of the present invention provides a composition for promoting angiogenesis including a cell-penetrating peptide.

Yet another aspect of the present invention provides a pharmaceutical composition for wound healing including a cell-penetrating peptide.

Yet another aspect of the present invention provides a pharmaceutical composition for preventing or treating angiogenesis-dependent diseases including a cell-penetrating peptide.

Yet another aspect of the present invention provides a method for wound healing including administering a cell-penetrating peptide to a wound in a pharmaceutically effective dose.

Yet another aspect of the present invention provides a method for treating angiogenesis-dependent diseases including administering a cell-penetrating peptide to a subject suffering from an angiogenesis-dependent disease in a pharmaceutically effective dose.

Yet another aspect of the present invention provides a use for preparing a composition for wound healing of a cell-penetrating peptide.

Yet another aspect of the present invention provides a use of for preparing a composition for preventing or treating angiogenesis-dependent diseases of a cell-penetrating peptide.

### [Advantageous Effects]

According to the present invention, cell-penetrating peptides have the effect of improving cell permeation, cell proliferation, cell migration and blood vessel formation, and thus can be used for cell-penetrating delivery, wound healing, and angiogenesis promotion. Particularly, the cell-penetrating peptides have an excellent effect of treating diabetic wounds (ulcers), which are intractable, and thus can be effectively used for wound healing.

### [Description of Drawings]

FIG. 1 is a diagram illustrating a structure of cell-penetrating peptide LPX.
FIG. 2 is a diagram confirming cell-penetrating characteristics of LPX101 peptide.
FIG. 3 is a diagram confirming cell-penetrating characteristics of LPX102 and LPX103 peptides.
FIG. 4 is a diagram confirming an effect of promoting cell growth and migration of LPX101 peptide.
FIG. 5 is a diagram confirming an effect of promoting cell migration of LPX101, LPX102, and LPX103 peptides.
FIG. 6 is a diagram confirming an angiogenic effect of LPX101 peptide.
FIG. 7 is a diagram confirming improvement in glucose uptake ability by LPX101, LPX102, and LPX103 peptides.
FIG. 8 is a diagram confirming improved glucose metabolism by LPX101, LPX102, and LPX103 peptides.
FIG. 9 is a diagram confirming a wound healing effect of LPX101 peptide in diabetes model mice.
FIG. 10 is a diagram illustrating histological analysis of a diabetic wound healing effect by LPX101 peptide.
FIG. 11 is a diagram confirming the acceleration of remodeling of the regenerated cortex by LPX101 peptide in diabetic wounds.
FIG. 12 is a diagram confirming an effect of improving wound healing ability by LPX101, LPX102, and LPX103 peptides in an STZ-induced diabetes mouse model.
FIG. 13 is a diagram comparing the wound regeneration activities of LPX101, LPX102, and LPX103 peptides in a diabetic mouse model (db/db).
FIG. 14 is a diagram of histological evaluation of wound healing and angiogenesis effects in a diabetic mouse model treated with LPX 103 or EGF.
FIG. 15 is a diagram illustrating histological evaluation of re-epithelialization by LPX101, LPX102 and LPX103 peptides.
FIG. 16 is a diagram confirming an effect of combined treatment of LPX101, LPX102, and LPX103 peptides, and EGF.
FIG. 17 is a diagram summarizing an operating method of LPX peptides to improve the regenerative effect in diabetic wounds.
FIG. 18 is a diagram illustrating amino acid sequences for LPX peptide variants.
FIG. 19 is a diagram illustrating amino acid sequences for LPX peptide variants.
FIG. 20 is a diagram illustrating sequences for LPX peptide variants of the present invention.
FIG. 21 is a diagram illustrating effects of LPX peptide variants (CPP region variants) on the stability of protein in *in-vitro* cultured cells, migration of cells, and proliferation of cells.
FIG. 22 is a diagram confirming an effect of LPX peptide variants (His tag addition variants) for endosomal escape on the stability of protein in cells, migration of cells, and proliferation of cells.
FIG. 23 is a diagram confirming the stability in cells of LPX peptide variants (RNA binding site and Zinc finger sequence variants).
FIG. 24 is a diagram confirming stabilization according to mutation of residues of screened LPX103 peptide.
FIG. 25 is a diagram confirming effects on cell proliferation and migration of variants of screened LPX103 peptide on stability of protein.
FIG. 26 is a schematic diagram illustrating changes in activity according to mutations in LPX peptide.
FIG. 27 is a diagram illustrating LPX105 peptides screened for improved biological activity.
FIG. 28 is a diagram confirming an effect of promoting cell migration according to mutation of LPX105 peptide.
FIG. 29 is a diagram confirming a diabetic wound healing effect according to mutation of LPX105 peptide (A: confirmation of reduction in diabetic wound area, B: confirmation of reduction in wound area on day 8).

### [Modes of the Invention]

Hereinafter, exemplary embodiments of the present invention will be described in detail. However, the following exemplary embodiments are presented as examples of the present invention, and the present invention is not limited thereby, and various modifications and applications of the present invention may be made within the description of claims to be described below and equivalents interpreted therefrom.

Unless defined otherwise, all technical and scientific terms used herein have the same meaning as commonly understood by those skilled in the art to which the present invention pertains. Although any methods and materials similar or equivalent to those described herein may be used in the practice for testing the present invention, the preferred materials and methods are described herein.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned in the present invention as abbreviations are also described as follows according to the IUPAC-IUB nomenclature.

Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

In one aspect, the present invention relates to a cell-penetrating peptide including a cell-penetrating domain and a zinc finger domain.

The zinc finger domain may include an amino acid sequence of Formula 1.
X1-X2-X3-X4-G-X5-L-D-X6-H-A-K-E-X7 (Formula 1, SEQ ID NO: 1)
Wherein,
X1 is C or A;
X2 is Y or R;
X3 is N, R or D;
X4 is C or A;
X5 is G or E;
X6 is H or A; and
X7 is C or A.

In one embodiment, the peptide may be a first zinc finger, and more preferably, X1 is C, X3 is N, X4 is C, X6 is H, and X7 is C.

In one embodiment, stability may be improved when X2 is R and X5 is E.

In one embodiment, the peptide may be CRNCGELDHHAKEC modified from CYNCGGLDHHAKEC to improve stability.

In one embodiment, the peptide of the present invention may include an amino acid sequence of the following Formula 2:
G-X8-R-C-X2-N-C-G-X5-L-D-H-H-A-K-E-C-K-X9 (Formula 2, SEQ ID NO: 2)
Wherein,
X8 is D or R;
X2 is Y or R;
X5 is G or E; and
X9 is L or W.

In one embodiment, if in Formula 2, X8 is R, X5 is E, and X9 is W, stability in cells may be improved.

In one embodiment, the peptide of Formula 2 may be a GDRCYNCGGLDHHAKECKL peptide of SEQ ID NO: 3, and here, there may be mutations D2R, C4A, Y5R, N6R, N6D, C7A, G9E, H12A, C17A and L19W, and the mutations D2R, Y5R, G9E, L19W improve stability, but the mutations C4A, N6R, N6D, C7A, H12A and C17A may have reduced stability.

In one embodiment, in the peptide of Formula 2 and SEQ ID NO: 2, X8, X5 and X9 are stability residues; 4th C, 7th C, 12th H and 17th C are zinc fingers; 3rd R, X2 and 15th K are affinity residues; and 6th N may be an RNA binding residue.

In one embodiment, an amino acid sequence represented by SEQ ID NO: 4 may be further included in an N-terminus (amino terminus), and more preferably, added to the N-terminus of the peptide of Formula 2 and SEQ ID NO: 2. The amino acid represented by SEQ ID NO: 4 may be a CPP domain, and may be changed to SEQ ID NO: 5 or 6.

In one embodiment, an amino acid sequence represented by SEQ ID NO: 5 may be further included in the N-terminus, and more preferably, added to the N-terminus of the peptide of Formula 2 and SEQ ID NO: 2.

In one embodiment, an amino acid sequence represented by SEQ ID NO: 6 may be may be further included in the N-terminus, and more preferably, added to the N-terminus of the peptide of Formula 2 and SEQ ID NO: 2.

In one embodiment, an amino acid E or GSE may be may be further included in the N-terminus, and more preferably, added to the N-terminus of the peptide of Formula 2 and SEQ ID NO: 2.

In one embodiment, a his tag may be further included in the N-terminus, may be 6-repeated 6XHis and more preferably, added to the N-terminus of the peptide of Formula 2 and SEQ ID NO: 2.

In one embodiment, an amino acid sequence represented by SEQ ID NO: 7 may be further included in a C-terminus (carboxyl terminus), and more preferably, added to the C-terminus of the peptide of Formula 2 and SEQ ID NO: 2.

In one embodiment, an amino acid sequence represented by SEQ ID NO: 8 may be further included in the C-terminus, and more preferably, added to the C-terminus of the peptide of Formula 2 and SEQ ID NO: 2. The amino acid sequence represented by SEQ ID NO: 8 may be a zinc finger (second zinc finger).

In one embodiment, an amino acid sequence represented by SEQ ID NO: 9 may be further included in the C-terminus, and more preferably, added to the C-terminus of the peptide of Formula 2 and SEQ ID NO: 2.

In one embodiment, an amino acid sequence represented by SEQ ID NO: 10 may be further included in the C-terminus, and more preferably, added to the C-terminus of the peptide of Formula 2 and SEQ ID NO: 2.

In one embodiment, the amino terminus and the carboxyl terminus of the peptide may be modified or protected with various organic groups to protect the peptide against protein-cleaving enzymes in vivo and increase stability, and for example, the C-terminus may be amidated.

In one embodiment, the peptide may consist of an amino acid sequence of any one represented by SEQ ID NOs: 37 to 40 (LPX105 and variants thereof).

In one embodiment, the peptide may be at least one peptide variant selected from the group consisting of LPX101 consisting of an amino acid sequence represented by SEQ ID NO: 11, LPX102 consisting of an amino acid sequence represented by SEQ ID NO: 12, LPX103 consisting of an amino acid sequence represented by SEQ ID NO: 13, and LPX104 consisting of an amino acid sequence represented by SEQ ID NO: 14.

In one embodiment, the LPX103 peptide variant may be a peptide variant consisting of an amino acid sequence selected from the group consisting of SEQ ID NO: 19, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, and SEQ ID NO: 36.

In one embodiment, the LPX104 peptide variant may be a peptide variant consisting of at least one amino acid sequence selected from the group consisting of SEQ ID NO: 15, SEQ ID NO: 16, SEQ ID NO: 17, SEQ ID NO: 18, SEQ ID NO: 20, SEQ ID NO: 21, SEQ ID NO: 22, and SEQ ID NO: 23.

In one embodiment, the cell-penetrating peptide may consist of an amino acid sequence represented by SEQ ID NO: 37, and may further include an amino acid sequence represented by SEQ ID NO: 38, an amino acid sequence represented by SEQ ID NO: 39, and an amino acid sequence represented by SEQ ID NO: 40.

The above-described protecting group serves to protect the peptide of the present invention from attack by protein-cleaving enzymes in vivo. In addition, the modified peptide exhibits excellent thermal stability due to the protecting group and also has excellent stability against physicochemical factors such as acid and alkali. Therefore, since the peptide of the present invention may be modified to have excellent long-term storage, the peptide may be advantageously applied to products that require long-term storage, such as pharmaceuticals, quasi-drugs, cosmetics, and oral care products.

The above-described amino acid modification (or variant or mutation) acts to greatly improve the stability of the peptide of the present invention, and the term "stability" referred to in the present invention refers not only to in vivo stability but also to storage stability (e.g., storage stability at room temperature).

In the present invention, even if the peptide is described as a `peptide consisting of a specific sequence number', if the peptide has the same or corresponding activity as a peptide consisting of the amino acid sequence of the sequence number, it is obvious that without excluding the addition of meaningless sequences before or after the amino acid sequence of the corresponding sequence number, mutations that may occur naturally, or silent mutations thereof, such sequence additions or mutations fall within the scope of the present application.

In the present invention, in the modification of the peptide sequence, some amino acids may be modified through any one of substitution, addition, deletion, and modification, or a combination of these methods. These modifications include modifications using L-type or D-type amino acids, and/or non-natural amino acids; and/or modifications by modifying a native sequence, such as modification of side chain functional groups, intramolecular covalent bonding, such as inter-side chain ring formation, methylation, acylation, ubiquitination, phosphorylation, aminohexation, biotinylation, etc. As the substituted or added amino acids, not only 20 amino acids conventionally observed in human proteins but also atypical or non-naturally occurring amino acids may be used. Commercial sources of the atypical amino acids include Sigma-Aldrich, ChemPep, and Genzyme pharmaceuticals. The sequences for the peptide including these amino acids and the typical peptide may be synthesized and purchased through commercialized peptide synthesis companies, such as American Peptide Company or Bachem in USA, or Anygen in Korea.

As used in the present invention, the term "peptide" refers to a linear molecule formed by binding amino acid residues with each other by peptide bonds. The peptides of the present invention may be prepared according to chemical synthesis methods known in the art, especially solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111 (1984)), and also produced by genetic manipulation techniques.

Specifically, the peptide of the present invention may be prepared by a standard synthetic method, a recombinant expression system, or any other methods in the art. Accordingly, peptides according to the present invention may be synthesized by many methods including, for example, the following methods:
(a) a method of synthesizing a peptide by stages or by fragment assembly by means of a solid phase or liquid phase method and isolating and purifying a final peptide product;
(b) a method of expressing a nucleic acid construct encoding the peptide in a host cell, and recovering an expression product from a host cell culture; or
(c) a method of performing the expression of the nucleic acid construct encoding the peptide within a cell-free tube, and recovering an expression product; or
a method of obtaining fragments of the peptide in any combination of (a), (b) and (c), and then linking the fragments to obtain a peptide, and recovering the corresponding peptide.

In a more specific example, a fusion gene encoding a fusion protein containing a fusion partner and the peptide of the present invention is prepared through genetic manipulation and transformed into a host cell, and expressed in a fusion protein form, and then the peptide of the present invention is cleaved and separated from the fusion protein using protease or a compound to produce the peptide of the present invention. To this end, for example, a DNA sequence encoding an amino acid residue that may be cleaved by protease such as Factor Xa or enterokinase and a compound such as CNBr or hydroxyl amine may be inserted between the fusion partner and a polynucleotide encoding the peptide of the present invention.

In one aspect, the present invention relates to a polynucleotide encoding the peptide of the present invention, a vector containing the polynucleotide sequence, or a host cell transformed with the vector.

As used in the present invention, the term "nucleotide" refers to deoxyribonucleotide or ribonucleotide that exists in a single-stranded or doublestranded form, and includes natural nucleotide analogs unless otherwise specified (Scheit, Nucleotide Analogs, John Wiley, New York(1980); Uhlman and Peyman, Chemical Reviews, 90:543-584(1990)).

As used in the present invention, the term "vector" refers to any nucleic acid including a competent nucleotide sequence that is inserted into a host cell to be recombined with a host cell genome and inserted to the host cell genome or replicates spontaneously as an episome. Such a vector includes a linear nucleic acid, a plasmid, a phagemid, a cosmid, an RNA vector, a viral vector, and the like.

As used in the present invention, the term "host cell" refers to a eukaryotic or prokaryotic cell into which one or more DNAs or vectors are introduced, and should be understood to refer to not only a specific target cell but also its progeny or potential progeny. Since a certain modification may occur in subsequent generations due to mutations or environmental influences, in fact, the progeny is not identical to a parent cell, but is still included within the scope of the term as used in the present invention.

In one aspect, the present invention relates to a composition for promoting cell proliferation and migration including the peptide of the present invention.

In one aspect, the present invention relates to a cell-penetrating carrier including the peptide of the present invention.

In one embodiment, the cell-penetrating carrier of the present invention may be used as a drug delivery system.

Typically, the cell-penetrating peptide may be connected to a cargo of interest through a non-covalent or covalent bond. In particular, it is preferable that the cell-penetrating peptide and the cargo of interest form a covalent conjugate through chemical cross-linking (Zatsepin, TS, et al., Curr. Pharm. Des., 11: 3639-3654(2005)). Accordingly, the peptide of the present invention may be covalently or non-covalently linked to the cargo of interest to be delivered into cells or tissues.

The term "cargo of interest" used in the present invention refers to a substance conjugated with the peptide of the present invention through a covalent or non-covalent bond to be transported into the cell, and includes, for example, chemicals, nanoparticles, peptides, polypeptides, antisense oligonucleotides, siRNA, shRNA, miRNA, and peptide-nucleic acid (PNA), but is not limited thereto. Additionally, when the cargo of interest is the polypeptide, the drug delivery system of the present invention may additionally include a linker between the cell-penetrating carrier containing the peptide of the present invention and the cargo of interest. As the linker used in the present invention, various linkers known in the art may be used. The linker may have a length and/or sequence specifically selected to maximize the activity, that is, cell-penetrating activity of the peptide of the present invention. Specifically, the linker is a linker consisting of a plurality of amino acid residues. The linker consisting of the amino acid sequence is described in Huston, et al., Methods in Enzymology, 203:46-88 (1991), and Whitlow, et al., Protein Eng., 6:989 (1993)), and the literature is incorporated herein by reference.

The drug delivery system of the present invention may be used for various purposes. Specifically, the drug delivery system of the present invention may be used to transport substances such as chemicals, nucleic acids, nanoparticles, etc., and the nucleic acid includes antisense oligonucleotide, siRNA, shRNA, miRNA, and PNA, but is not limited thereto. In addition, the drug delivery system of the present invention may be used for the treatment of diseases or disorders, detection of specific cells, diagnosis of diseases (e.g., cancer), location tracking of specific cells, and in vivo imaging, etc., depending on a cargo to be delivered. The term "nucleic acid molecule" used in the present invention is meant to comprehensively include DNA (gDNA and cDNA) and RNA molecules, and a nucleotide, which is a basic structural unit in the nucleic acid molecule, may include not only a natural nucleotide but also analogues with modified sugar or base sites (Scheit, Nucleotide Analogs, John Wiley, New York(1980); Uhlman and Peyman, Chemical Reviews, 90:543-584(1990)).

In addition, various labeling substances may be used for the peptide and the cargo of interest constituting the drug delivery system of the present invention, and include, for example, fluorescent substances [e.g., fluorescein, fluoresein isothiocyanate (FITC), rhodamine 6G, rhodamine B, 6-carboxy-tetramethylrhodamine (TAMRA), Cy-3, Cy-5, Texas Red, Alexa Fluor, 4,6-diamidino-2-phenylindole (DAPI), and Coumarin], fluorescence proteins (GFP, RFP, CFP, YFP, BFP, luciferase or variants thereof), radioactive isotopes (e.g., C¹⁴, I¹²⁵, P³² and S³⁵), chemicals (e.g., biotin), luminescent substances, chemiluminescent and fluorescence resonance energy transfer (FRET). When the drug delivery system of the present invention is configured by including radioactive isotopes (e.g., configured by the peptide of the present invention and nanoparticles), the drug delivery system may also be used for tissue imaging by applying single photon emission computed tomography (SPECT) or positron emission tomography (PET).

In one aspect, the present invention relates to a composition for tissue regeneration including the peptide of the present invention.

In one aspect, the present invention relates to a composition for promoting angiogenesis including the peptide of the present invention as an active ingredient.

In one aspect, the present invention relates to a pharmaceutical composition for wound healing including the peptide of the present invention as an active ingredient.

In one embodiment, the wound may be non-healing traumatic wound, destruction of tissue by irradiation, abrasion, bone gangrene, laceration, avulsion, penetrated wound, gunshot wound, cuts, frostbite, contusion or bruise, skin ulcers, dry skin, skin keratosis, cracking, bursting, dermatitis, pain due to dermatophytosis, wounds such as surgical wound, vascular disease wound and corneal wound, bedsores, decubitus, conditions related to diabetes such as diabetic skin erosion and poor circulation, chronic ulcers, suture sites after plastic surgery, spinal injury wound, gynecological wound, chemical wound or acne.

In one aspect, the present invention relates to a pharmaceutical composition for preventing or treating angiogenesis-dependent diseases including the peptide of the present invention as an active ingredient.

In one embodiment, the angiogenesis-dependent disease may be at least one selected from the group consisting of ischemic disease, wound, burns, psoriasis, chronic ulcer, myocardial infarction, angina, bedsores, hair loss, diabetic retinopathy, retinopathy of prematurity, age-related macular degeneration, glaucoma, diabetic ulcer, pulmonary hypertension and cerebrovascular dementia.

In one embodiment, the ischemic disease may be at least one selected from the group consisting of cerebral ischemia, cardiac ischemia, diabetic vascular heart disease, heart failure, myocardial hypertrophy, retinal ischemia, ischemic colitis, ischemic acute renal failure, stroke, brain trauma, and neonatal hypoxia.

In the present invention, the term "prevention" refers to any action that inhibits or delays the occurrence, spread, and recurrence of angiogenesis-dependent diseases by administration of the protein or a fragment thereof according to the present invention, or a composition including the same.

The therapeutically effective dose of the composition of the present invention may vary depending on many factors, for example, an administration method, a target site, a condition of a patient, and the like. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective dose. In the present invention, the 'pharmaceutically effective dose' refers to an amount enough to treat the disease at a reasonable benefit/risk ratio applicable to medical treatment and not cause side effects. An effective dose level may be determined according to factors including the health condition, the type and severity of a disease of a patient, the activity of a drug, the sensitivity to a drug, a method of administration, a time of administration, a route of administration, an excretion rate, duration of treatment, and combination or simultaneously used drugs, and other factors well-known in the medical field. The composition of the present invention may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may include a carrier, diluent, excipient, or a combination of two or more thereof, which are commonly used in biological agents. As used in the present invention, the term "pharmaceutically acceptable" means that the composition exhibits non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be formulated in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be preferably formulated according to each disease or ingredient using as a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by, for example, compounds described in Merck Index, 13th ed., Merck & Co. Inc., saline, sterile water, a Ringer's solution, buffered saline, a dextrose solution, a maltodextrin solution, glycerol, ethanol, and mixtures of one or more of these ingredient, and if necessary, other conventional additives such as an antioxidant, a buffer, and a bacteriostat may be added. In addition, the pharmaceutical composition may be formulated in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be formulated preferably according to each disease or ingredient using as a suitable method in the art or a method disclosed in Remington's Pharmaceutical Science (Mack Publishing Company, Easton PA, 18th, 1990).

The composition of the present invention may further contain one or more active ingredients exhibiting the same or similar function. The composition of the present invention includes 0.0001 to 10 wt%, preferably 0.001 to 1 wt% of the protein, based on the total weight of the composition.

The pharmaceutical composition of the present invention may further include a pharmaceutically acceptable additive. At this time, the pharmaceutically acceptable additive may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present invention is preferably included in an amount of 0.1 part by weight to 90 parts by weight based on the composition, but is not limited thereto.

The composition of the present invention may be administered parenterally (e.g., intravenously, subcutaneously, intraperitoneally or topically) or orally according to a desired method, and the dose may vary depending on the weight, age, sex, and health condition of a patient, a diet, an administration time, an administration method, an excretion rate, and the severity of a disease. A daily dose of the composition according to the present invention is 0.0001 to 10 mg/ml, preferably 0.0001 to 5 mg/ml, and more preferably administered once to several times a day.

Liquid formulations for oral administration of the composition of the present invention correspond to suspensions, internal solutions, emulsions, syrups, etc., and may include various excipients, such as wetting agents, sweeteners, fragrances, preservatives, and the like in addition to water and liquid paraffin, which are commonly used simple diluents. Formulations for parenteral administration include sterilized aqueous solutions, non-aqueous solvents, suspensions, emulsions, lyophilized agents, suppositories, and the like.

### [Modes of the Invention]

Hereinafter, the present invention will be described in more detail with reference to the following Examples. However, the following Examples are only intended to embody the contents of the present invention, and the present invention is not limited thereto.

### Example 1. Confirmation of characteristics of LPX peptide

### 1-1. Cell penetrating effect

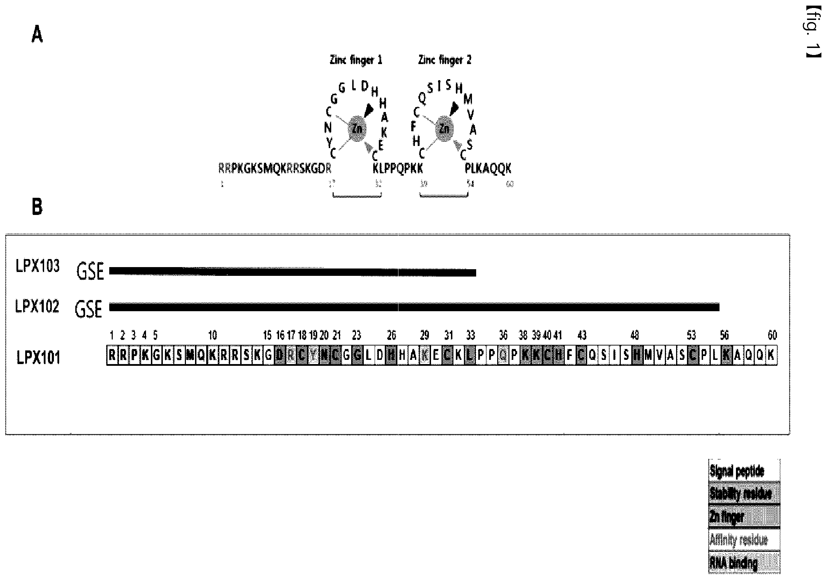

A cell penetrating effect was confirmed in penetrating peptides LPX101 (SEQ ID NO: 11), LPX102 (SEQ ID NO: 12), and LPX103 (SEQ ID NO: 13) having three structures including a signal peptide, an RNA binding affinity residue, and an RNA binding residue (FIG. 1), and two zinc finger motifs. Specifically, in order to measure cell penetrating efficiency, respective peptides that may have been attached to the cell membrane surface were removed by applying a Heparin & Trypsin washing method used in a Keplan group [Kaplan IM, Wadia JS, Dowdy SF (2005) Cationic TAT peptide transduction domain enters cells by macropinocytosis. J Controlled release 102: 247-253] to a NIH3T3 cell line and mouse mesenchymal stem cells (mMSCs). Thereafter, LPX101 (20 µg/ml), LPX103, and LPX102 (0, 0.1, 1, 2, 4 µg/ml) peptides were synthesized in the form attached with FITC by LifeTein (USA), and then each added in a medium growing each cell at a concentration of 1 µM and incubated at 37°C for 1 hour. Then, the cells were washed three times with PBS and detached with trypsin, and then FACS analysis was performed. In addition, whether each peptide actually existed in the nucleus or cytoplasm of the cell was confirmed by confocal microscopy after 18 hours of peptide treatment, and membrane binding was labeled with PKH.

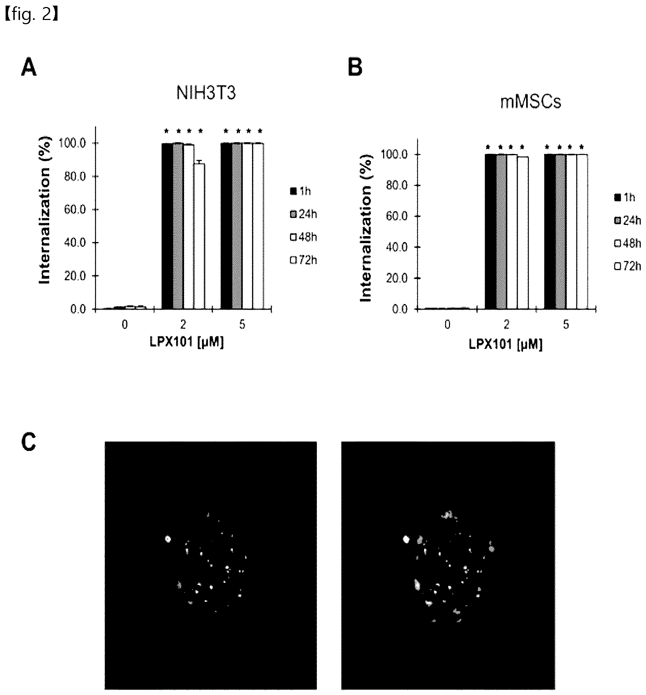

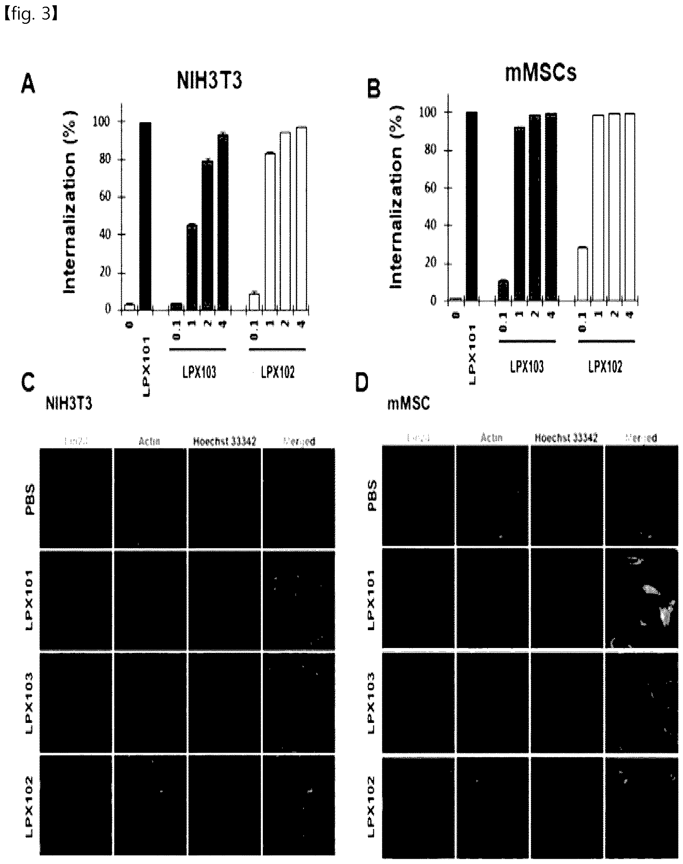

As a result, LPX101 showed high cell permeability at both concentrations of 2 µM and 5 µM, and it was confirmed that most of LPX101 was present in the cytoplasm (FIG. 2). In addition, both LPX102 and LPX103 were confirmed to have similar intracellular retention at a concentration of 2 µM or higher, in addition to high cell permeability similar to LPX101 (2 µM) (FIG. 3).

### 1-2. Effect of promoting cell growth and migration

An effect of LPX101 peptide on cell growth was confirmed in a NIH3T3 cell line, and the effects of LPX101, LPX102, and LPX103 peptides on cell migration were confirmed in diabetic human epidermal keratinocyte (DHEK) as a diabetic cell line. Specifically, mMSC or NIH3T3 cells were treated with the LPX101 peptide, incubated for 1 hour, and then washed. A % increase in cell growth by LPX treatment was confirmed by measuring the number of cells after 3 days of treatment and calculating a relative proliferation rate as % compared to a control group (n = 3). Additionally, in order to confirm the cell migration effect, DHEK cells were treated with LPX101, LPX102, and LPX103 peptides, incubated for 1 hour, and then washed. Thereafter, the migration of cells across a scratched boundary was measured 72 hours after treatment. The number of cells that migrated across a scratch line was measured by image analysis using ImageJ.

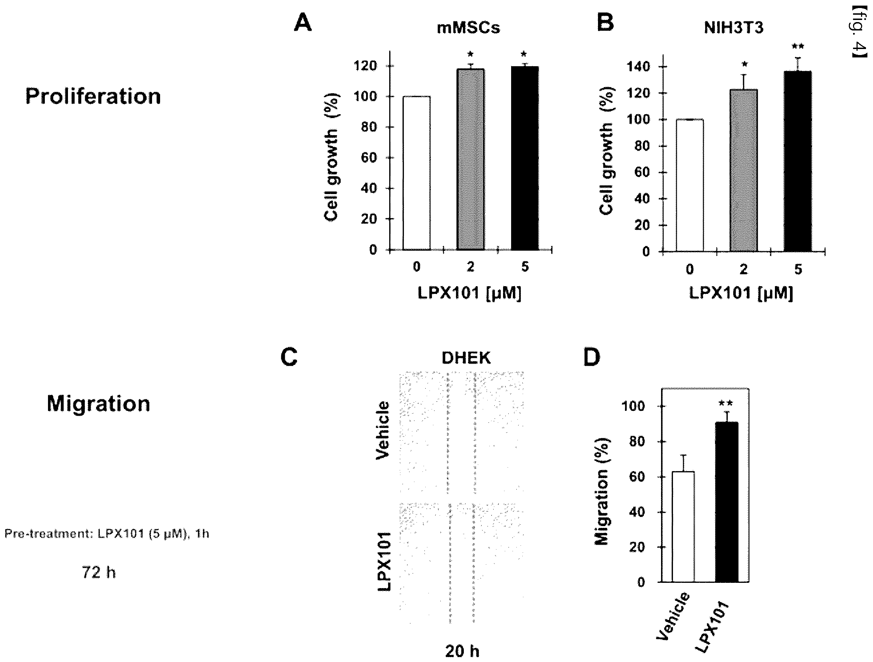

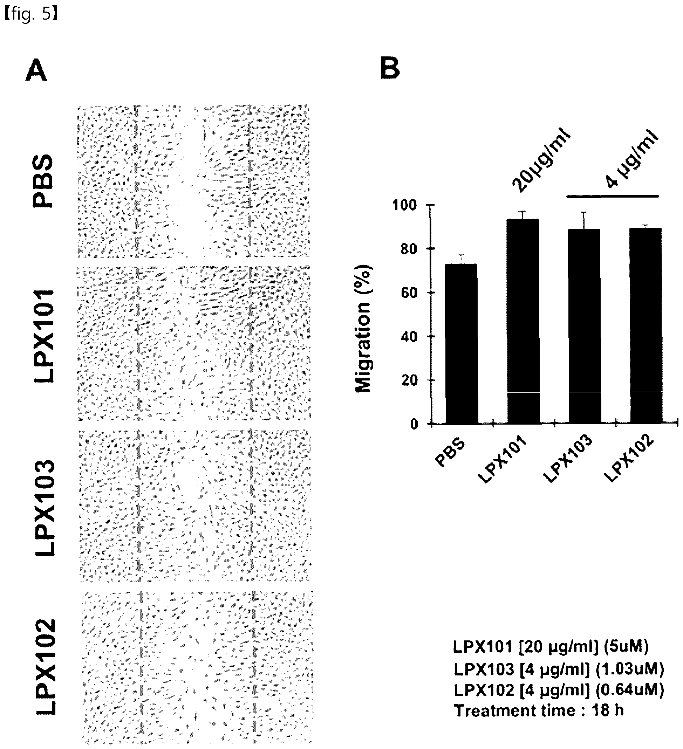

As a result, it was shown that the LPX101 peptide promoted cell growth at both concentrations of 2 µM and 5 µM and promoted the migration of DHEK cells (FIG. 4). Additionally, LPX102 and LPX103 peptides promoted cell migration to a similar extent to LPX101 at lower concentrations (FIG. 5).

### 1-3. Effect of promoting blood vessel formation

Human umbilical vein endothelial cells (Huvec) were dispensed on a cytokine-free Matrigel and treated with LPX101 for one day (n = 3, 1 expt). After 19 days, the blood vessel formation was confirmed under a microscope, and blood vessel formation parameters including the number of nodules, the number of intravascular junctions, and the number of master segments were quantified.

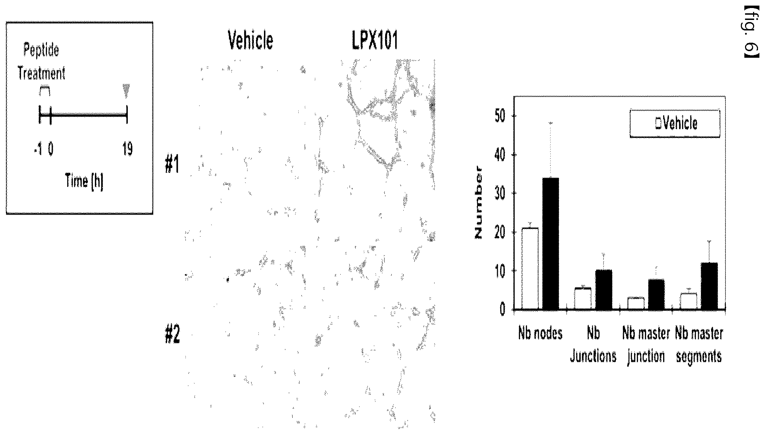

As a result, endothelial cells appeared to form more mature capillary vascular rings by treatment with the LPX101 peptide (FIG. 6).

### Example 2. Confirmation of anti-diabetic effect of LPX peptide

### 2-1. Effect of improving glucose uptake ability

Human dermal fibroblasts (HDF) and a diabetic cell line DHEK were incubated in a glucose-free medium for 24 hours, treated with 2 µM (20 µg/ml) of LPX101, 1 µM of LPX102, or 1 µM of LPX103 for 18 hours, and then treated with 2-deoxyglucose (2DG). The amount of absorbed glucose reacted with a luminiscemce kit (promega) and then measured at 470 nm using Spectra MaxL equipment to measure a relative glucose uptake (mean SEM, n = 3, *;p < 0.05, **: p < 0.01).

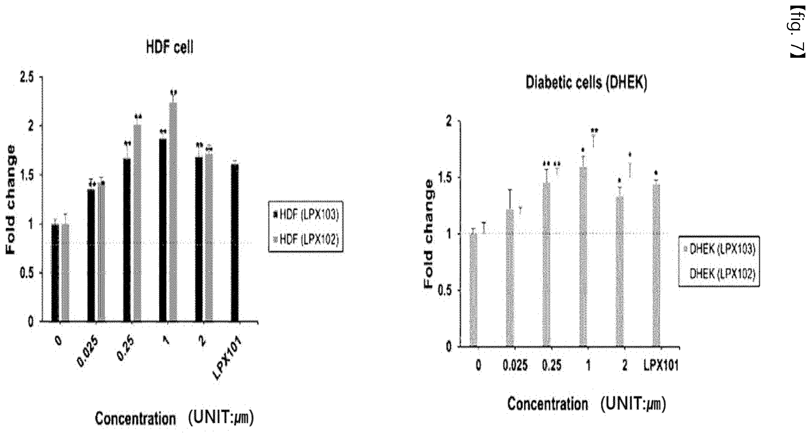

As a result, it was shown that the LPX peptides improved glucose uptake in several mammalian cells, including a diabetic cell line (FIG. 7).

### 2-2. Effect of improving glycolysis

NIH3T3 cells were incubated in a glucose-free medium for 1 day and then sequentially treated with glucose, oligomycin, and 2-DG, and an extracellular acidification rate (ECAR) was measured as a measure of lactate production using a Seahorse extracellular flux (XF) analyzer and quantification and profile of ECAR were confirmed in cells treated with LPX peptide (Mean SEM n = 3, *; p < 0.05).

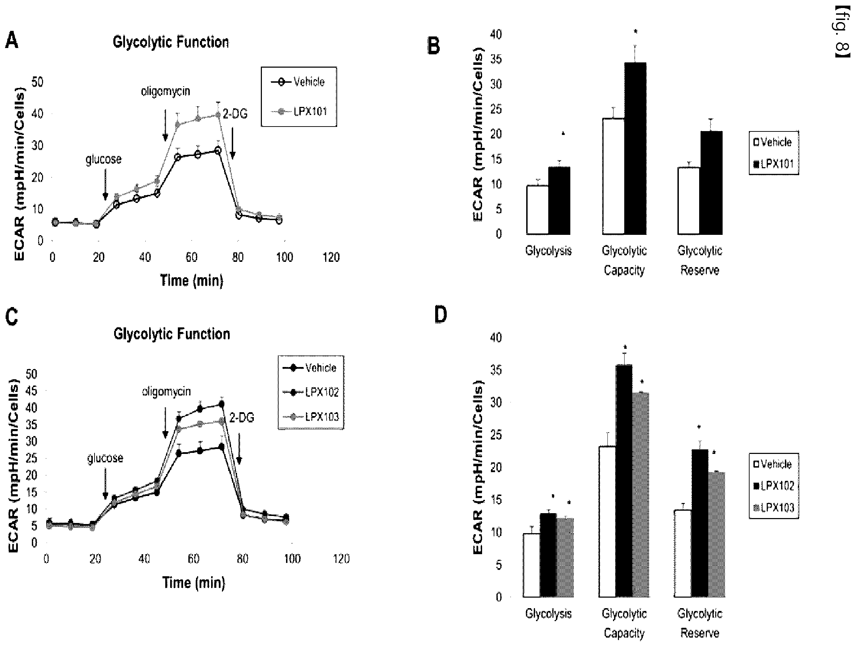

As a result, it was shown that LPX101, LPX102 and LPX103 peptides promoted glucose metabolism (glycolysis) (FIG. 8).

### 2-4. Diabetic wound healing effect

### 2-4-1. Confirmation of diabetic wound healing effect

The skins of wild type (WT) mice and diabetes model mice (db/db) (n = 14 each) were punched with an 8 mm diameter puncher and treated with LPX101 (2 mg/ml, 20 µl) or PBS daily for 14 days and subjected to wound dressing. The wound healing effect was confirmed by analyzing an initial wound area and a re-epithelialization area using ImageJ (mean SEM n = 3, *; p < 0.05). In addition, the wound healing effect in wounds treated with LPX101 was histologically evaluated. Specifically, diabetic mice (db/db) were stained with H/E or trichrome at 7 and 14 days after wound induction and then confirmed under a microscope, and re-epithelialization and granulation tissue were quantified using ImageJ (n = 3, ***; p < 0.001). In addition, the wounds of db/db mice were treated with LPX101, and the tissue epidermis after 14 days was histologically analyzed.

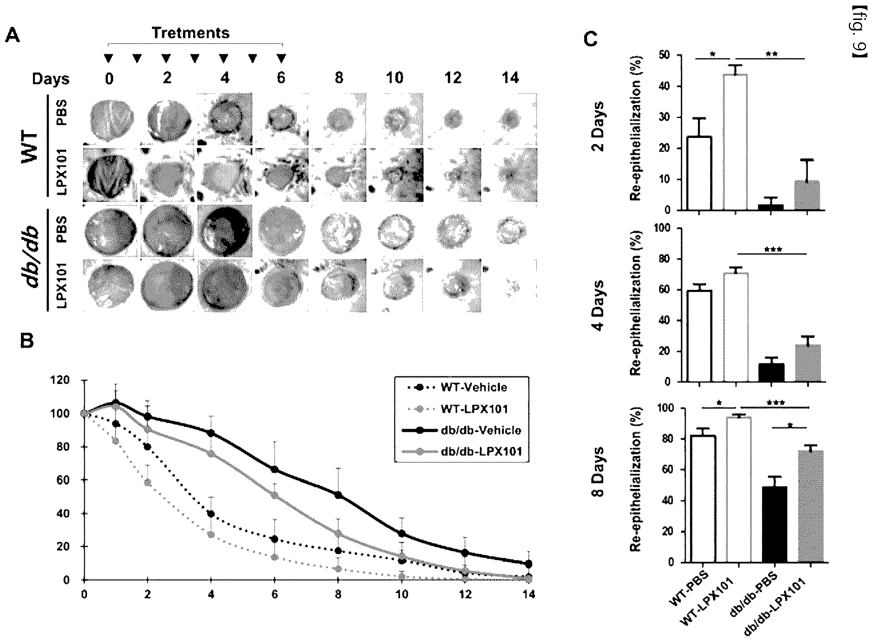

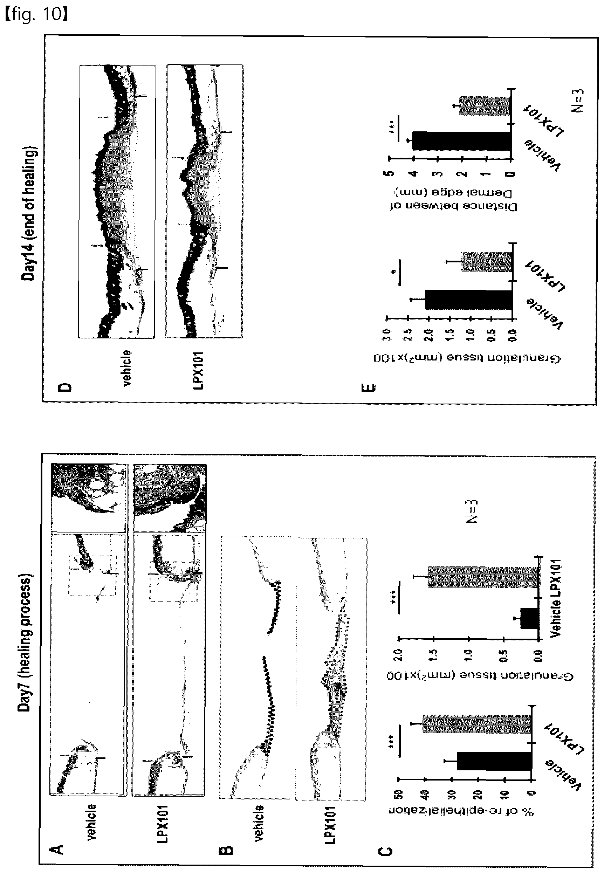

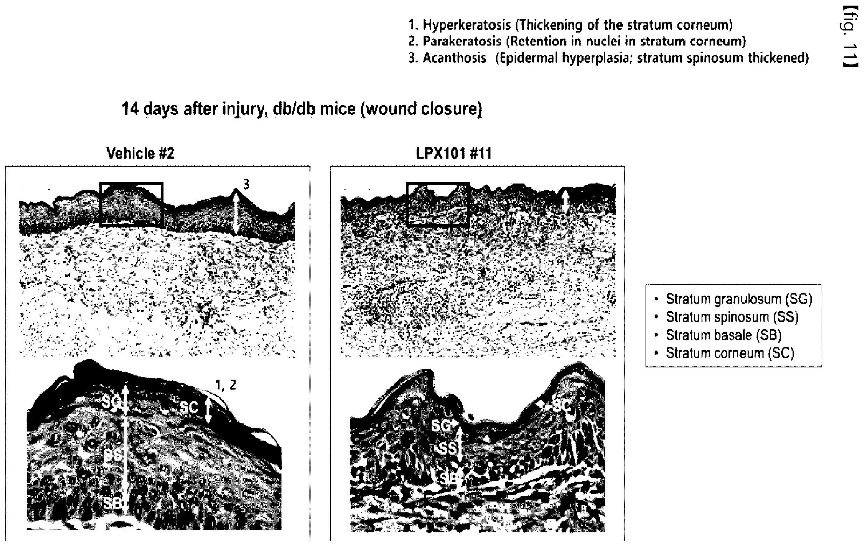

As a result, the LPX101 peptide accelerated wound healing in WT mice and significantly accelerated the healing of diabetic wounds in the db/db mouse model (FIG. 9). Additionally, it was shown that wounds induced in a diabetic mouse model due to treatment with LPX101 peptide caused early formation of granulation tissue on day 7 (in the middle of the healing process), and caused early shrinkage (remodeling) of granulation tissue on day 14 (end of the healing process) (FIG. 10). In addition, as the result of histological analysis of the epidermis on day 14, it was shown that hyperkeratosis, parakeratosis, and acanthosis were reduced early by LPX101 treatment, and remodeling of the regenerated wound epithelium was accelerated (FIG. 11).

### 2-4-2. Wound healing effect in STZ-induced diabetic mouse model

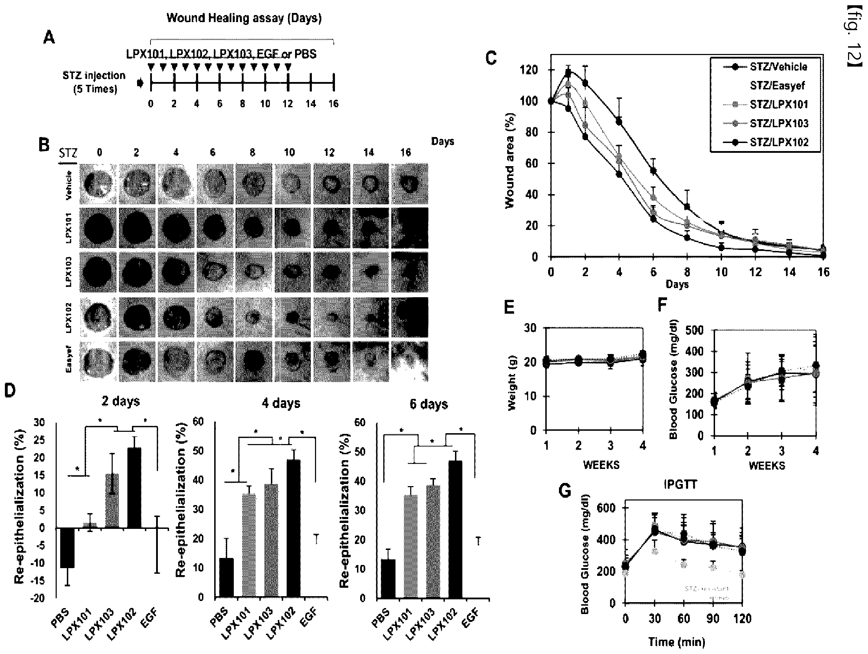

The wound healing effects of LPX101, LPX102, and LPX103 were confirmed in an STZ-induced diabetic mouse model. Specifically, diabetes was induced in mice by injecting streptozotocin (STZ) for 5 days, which resulted in hyperglycemia. Thereafter, according to a schedule shown in FIG. 12A, PBS (negative control), EGF (positive control) 50 µg/ml, LPX101 2 mg/ml (20 µl), LPX102 and LPX103 (2 mg/ml (20 µl)) were treated, respectively, and then the effect of wound healing in a diabetes-induced mouse model was confirmed through imaging, re-epithelialization, and diabetes phenotypes (hyperglycemia and impaired glucose tolerance test (IPGTT)) (n = 10 for each group, *; p < 0.05). Additionally, to compare the regenerative activity of LPX peptides in a diabetic mouse model, wound healing kinetics measured by a % non-epithelialized area to an initial wound area (n = 8 for each group), and quantification of re-epithelialization analyzed as % of a re-epithelialized area to the initial wound area were confirmed.

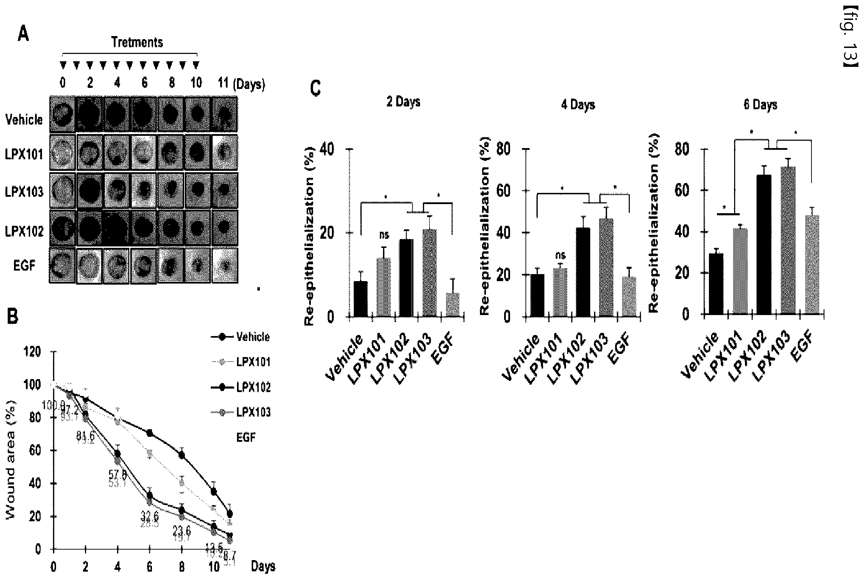

As a result, it was shown that the LPX peptides all had an improved wound healing effect compared to EGF in the STZ-induced diabetic wound model (selection of mice with obvious hyperglycemia), and particularly, LPX102 and LPX103 showed better effects than LPX101 (FIG. 12). In addition, even in the result of quantifying wound healing kinetics and re-epithelialization, it was shown that LPX102 and LPX103 had a better diabetic wound healing effect than LPX101 in the db/db mouse model (FIG. 13).

### 2-4-3. Diabetic wound healing effect by promoting blood vessel formation

Since diabetic ulcers were hardly treated by insufficient vascular supply due to impaired angiogenesis and ischemic necrosis, it was confirmed whether the LPX peptide contributed to blood vessel formation in diabetic wound healing. Specifically, diabetic model mice (db/db) were wounded and then treated with LPX 103 or EGF once daily, and the recovery of angiogenesis was analyzed on day 7 after wounding, and in order to visualize the extravasation of red blood cells within the tissue, the wound and entrance (red dotted box) of each group were magnified and images were confirmed.

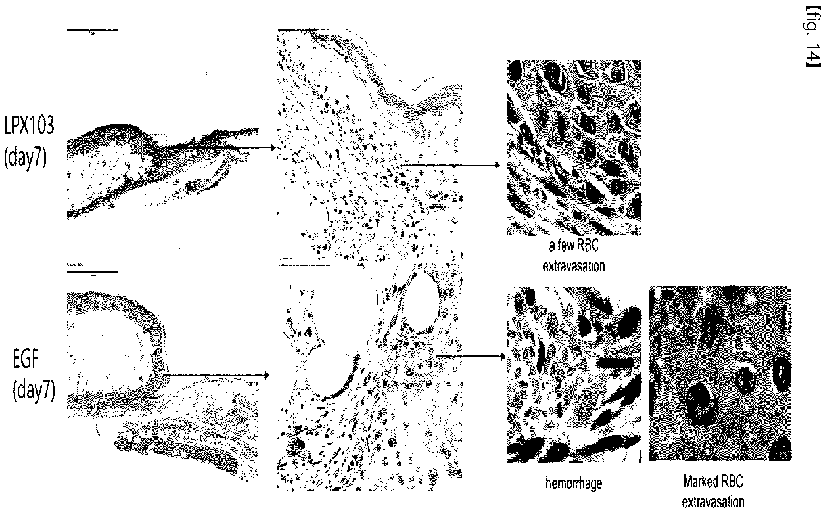

As a result, the group treated with LPX103 showed more mature capillary formation and less hemorrhage (extravasation of RBC) than a group treated with EGF (FIG. 14).

### 2-4-4. Histological evaluation of re-epithelialization by LPX peptide

In order to histologically evaluate wound re-epithelialization in diabetic mice by LPX peptides 101, 102 and 103, wound tissues from diabetic mice (db/db) treated by each LPX were H/E stained on day 10 after initial injury and confirmed under a microscope (50X and 100×).

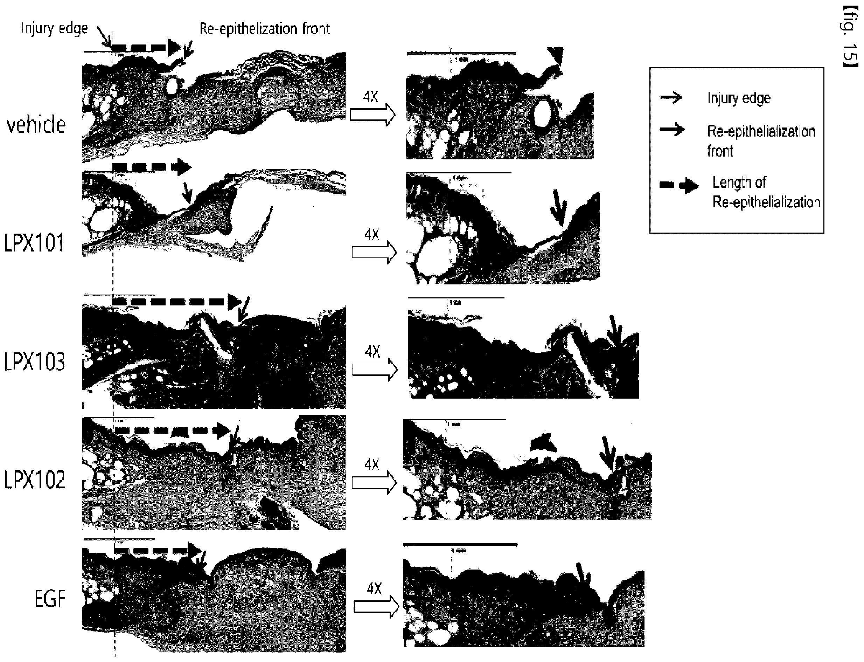

As a result, it was shown that LPX peptides 101, 102, and 103 all significantly improved re-epithelialization of diabetic wounds (FIG. 15).

### 2-4-5. Combined administration

The diabetic wound healing effect by combined treatment with LPX peptide and EGF was confirmed. Specifically, a diabetic mouse model (db/db) was wounded by punching at an 8 mm diameter and treated with a combination of LPX peptide (101, 102, or 103) and/or EGF as shown in FIG. 15A, and the images thereof were observed under a microscope, and the effect on re-epithelialization after initial wound development was confirmed.

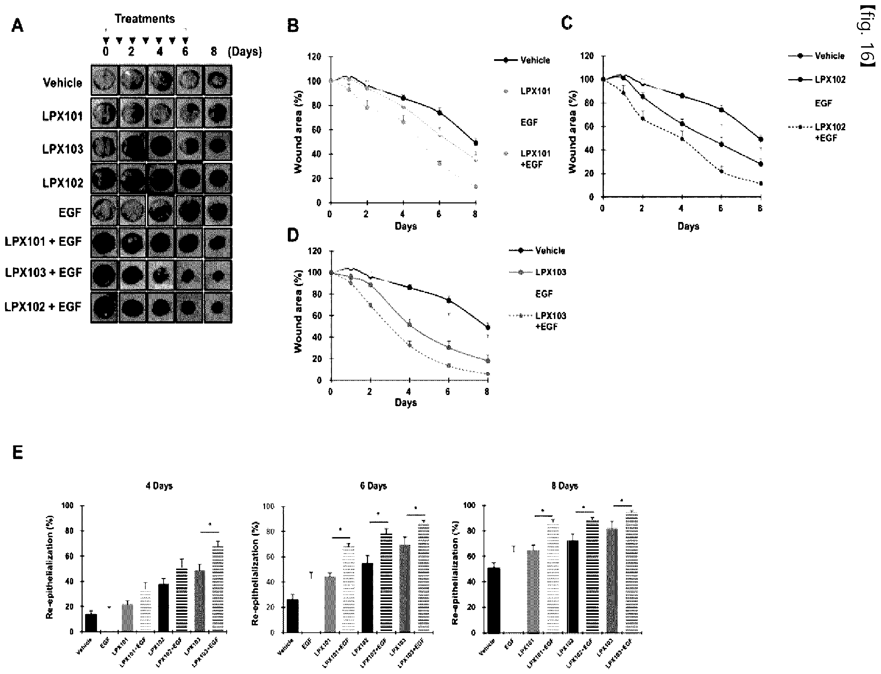

As a result, it was shown that each diabetic wound healing effect of LPX101, LPX102, and LPX103 was improved when treated in combination with EGF (FIG. 16).

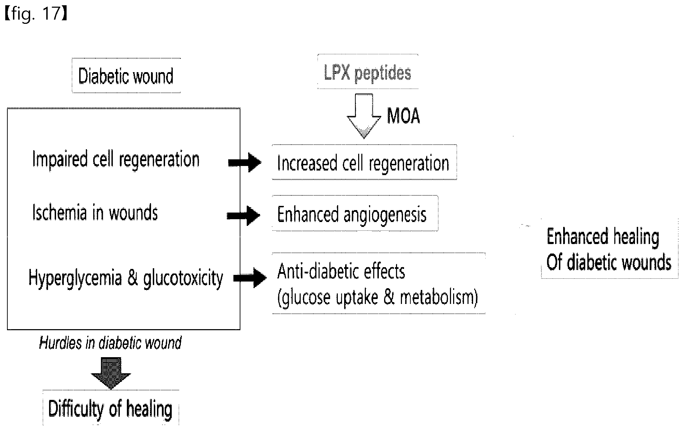

Through this, it was found that the LPX peptides multi-layeredly improved the regeneration of diabetic wounds (ulcers), which were previously difficult to be treated (FIG. 17).

### Example 3. Confirmation of activity according to modification of LPX peptide

### 3-1. Preparation of peptide variants

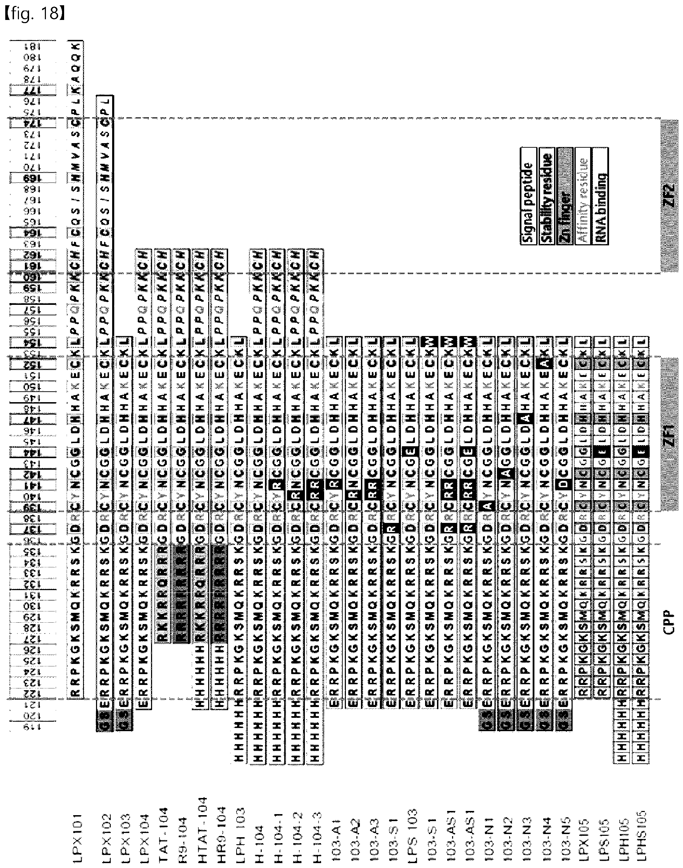

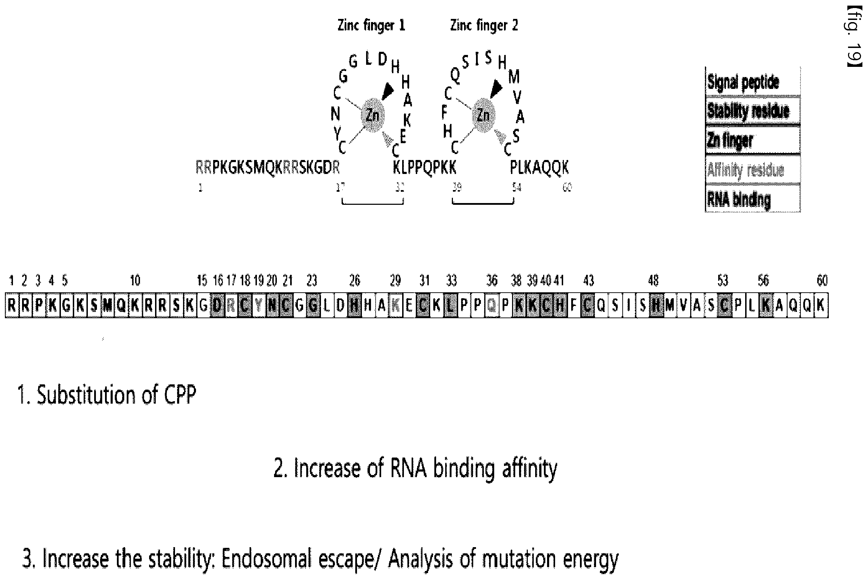

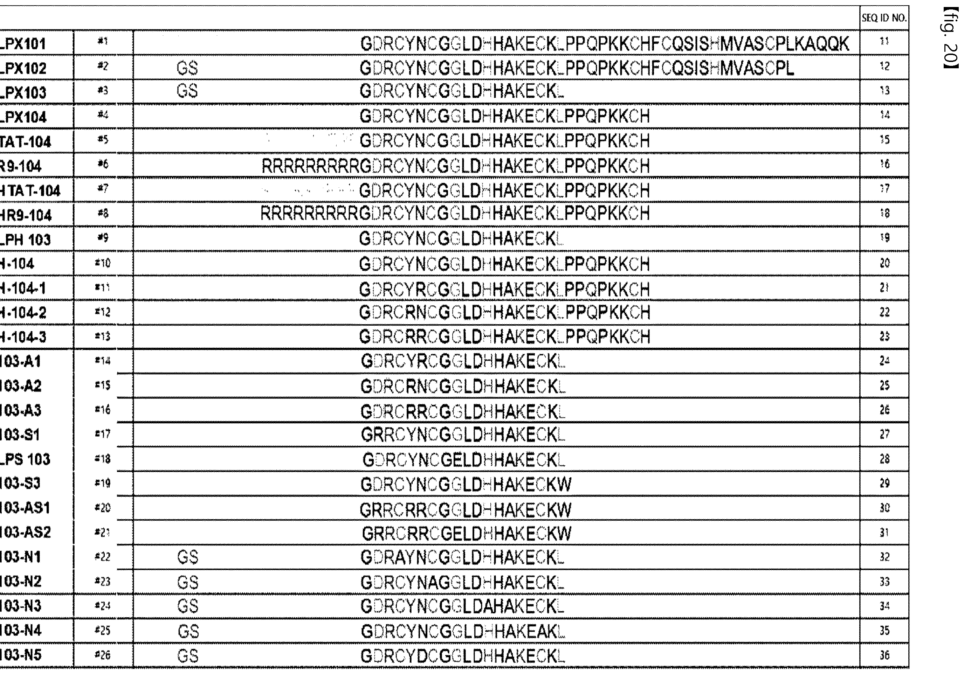

Various variants of the LPX peptides were prepared as illustrated in FIG. 18. Specifically, various constructs (26 constructs including 101) were prepared by adding various mutations (substitution of a CPP region, amino acid mutation of an RNA binding portion and a zinc finger structure, and addition of his-tag) to the structure of LPX101 in FIG. 19 (FIG. 20). Additionally, as a result of comparing the cell migration effects of LPX101, LPX102, LPX103 and LPX104 (SEQ ID NO: 14) on NIH3T3 cells, the cell migration effects were found to be similar to each other as shown in Table 1 below.

**[Table 1]**

| Effects on cell migration (3 expts) | | | | | |
|---|---|---|---|---|---|
| | vehicle | LPX101 | LPX102 | LPX103 | LPX104 |
| SuM | 1 | 1.3 | 1.2 | 1.3 | 1.3 |
| 1uM | 1 | 1.4 | 1.2 | 13 | 13 |

### 3-2. Confirmation of activity changes according to CPP region substitution

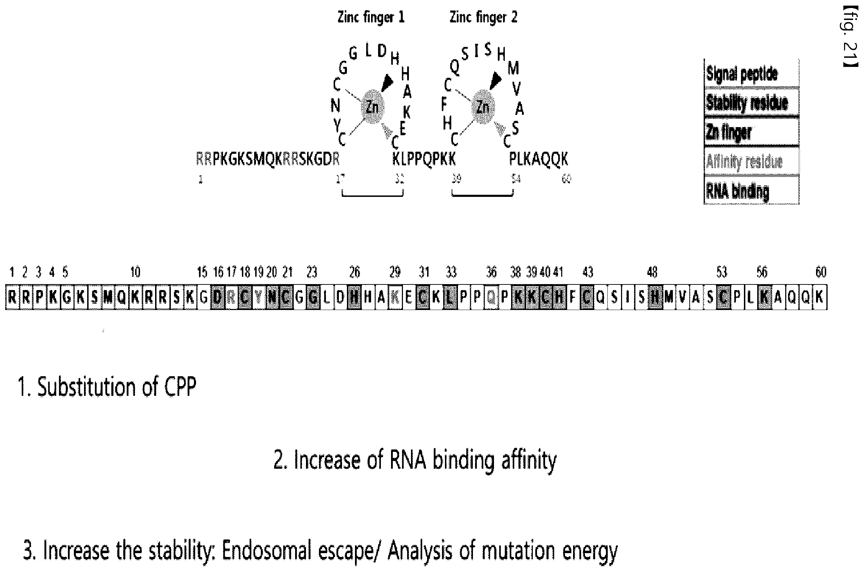

In order to confirm changes in biological activity due to substitution of the CPP region sequence, as illustrated in FIG. 21A, the CPP region was substituted with a TAT or Arg repeat (R9) sequence of HIV in LPX104 (#4) containing SEQ ID NO: 7, which included an RNA binding site in a zinc finger 2 region (each of TAT-104 and R9-104) and then for NIH3T3 cells, the cell penetration effect, the stability of the peptide after 24 hours of cell penetration, the cell migration effect, and the cell growth effect were compared.

As a result, substitution of the CPP region in the LPX peptide with TAT or Arg (9) residues showed similar effects to the original LPX104 on cell invasion, cell proliferation, and cell migration (FIG. 21B), and the stability of the peptide was significantly higher than that of the existing LPX104 (FIG. 21C).

### 3-3. Confirmation of activity changes according to HIS tag insertion

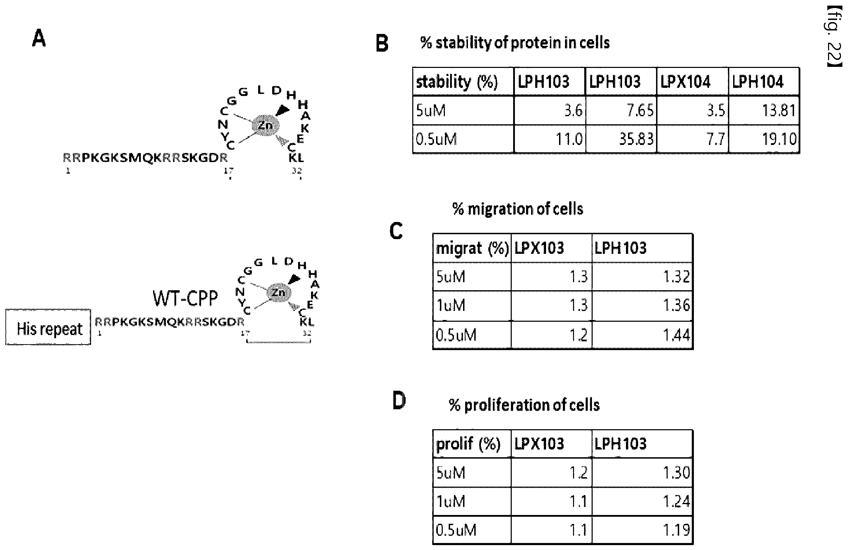

In order to select mutations with improved stability through endosomal escape, as illustrated in FIG. 22A, a repeated HIS tag sequence (6XHis) was added to the N-terminus of the CPP region of the LPX peptide (LPH103 or LPX104) and the stability, the cell migration promotion effect, and the cell growth promotion effect after 24 hours of cell penetration were compared.

As a result, it was shown that when the 6XHis tag was added to the LPX peptide, intracellular stability was significantly increased by helping endosomal escape (FIG. 22B). In addition, it was shown that the cell proliferation and cell migration effects were increased (FIGS. 22C to 22D).

### 3-4. Confirmation of activity changes according to mutations in RNA binding region and zinc finger region

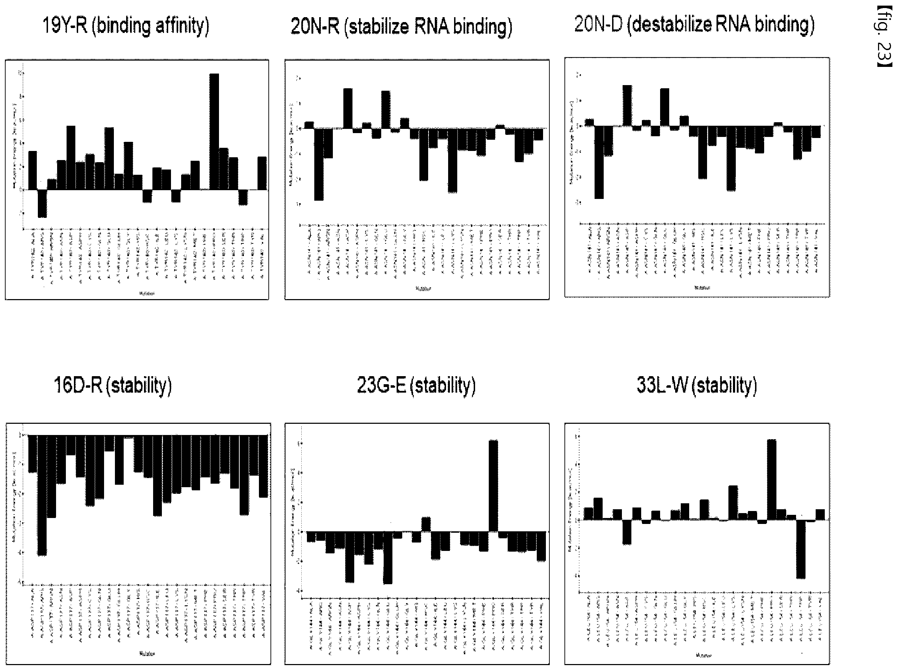

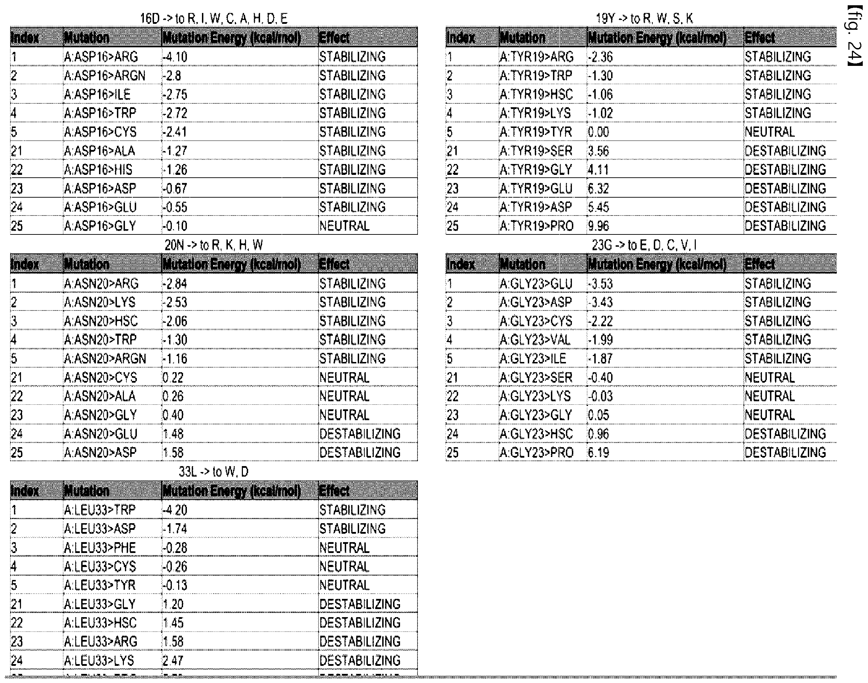

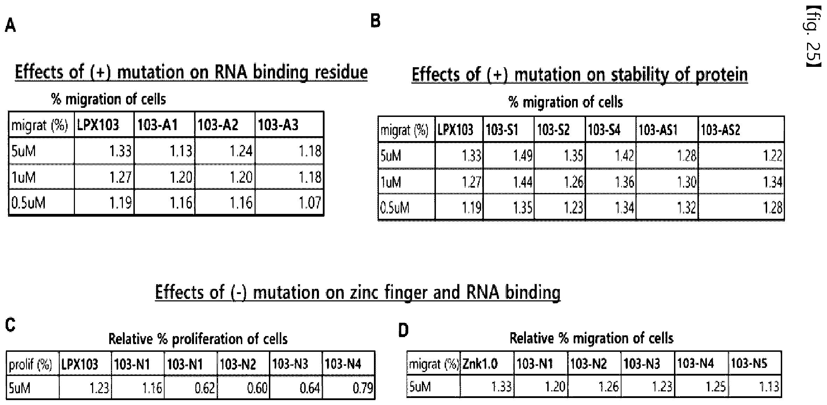

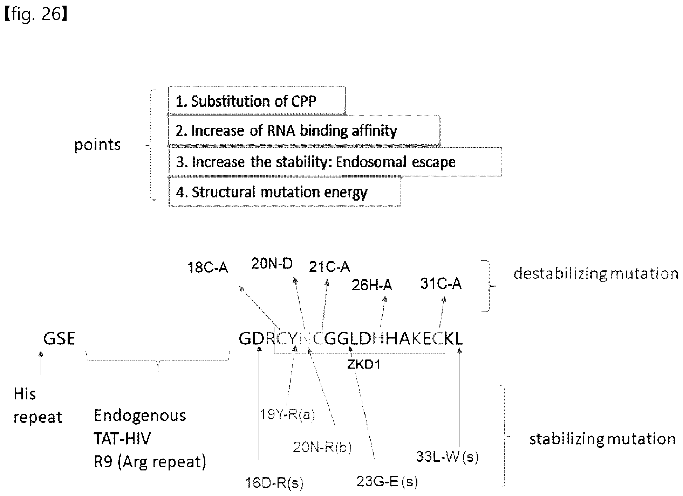

The protein stability of variants with mutations in the RNA binding region and the zinc finger region was confirmed. Specifically, LPX103 variants with mutated amino acid residues for RNA binding during cell migration were treated with NIH3T3 cells for 1 day, respectively, and based on the molecular modeling program, the intracellular stabilization effect of each peptide was analyzed after 72 hours. The mutation energy of each amino acid residue was confirmed, and variants that may cause <-1 Kcal/mol due to substitution were selected as potential mutations that may increase the stability and activity of the peptide (FIGS. 23 and 24). The effects of the selected variants on cell proliferation and cell migration were confirmed in NIH3T3 cells (FIG. 25), and as a result, mutations in the RNA binding region and the zinc finger region that improved or decreased the stability of the peptide in the cell were confirmed (FIG. 26). Accordingly, LPX103, LPH103, and LPS103, which were confirmed to have high stability and biological activity, were selected.

Through Examples, potential substitutions of residues capable of similarly maintaining or improving the biological activity of the LPX peptide were as follows:
1) addition of a histidine repeat sequence at the N-terminus improved stability;
2) substitution of CPP with TAT or R9 had equivalent effect;
3) residue mutations that caused stabilization of the protein (mutation energy <-1 Kcal/mole) were D16R, Y19R, N20R, G23E, L33E and L33W (see FIG. 26); and
4) Random combination of residues derived from different categories.

Additionally, the following residues that may cause a decrease in biological activity should be preserved in LPX peptide variants:
1) 18C, 21C, 26H and 31C of zinc finger motif (based on LPX101 of SEQ ID NO: 11); and
2) 20N of RNA binding region (based on LPX101 of SEQ ID NO: 11).

Through this, amino acid residues that may be replaced (substituted) or added because the stability and biological activity of LPX peptide were equalized or increased and residues that should be preserved because the stability and biological activity were decreased were identified.

### Example 4. Confirmation of activity according to modification of LPX105 peptide

### 4-1. Preparation of LPX105 peptide variants

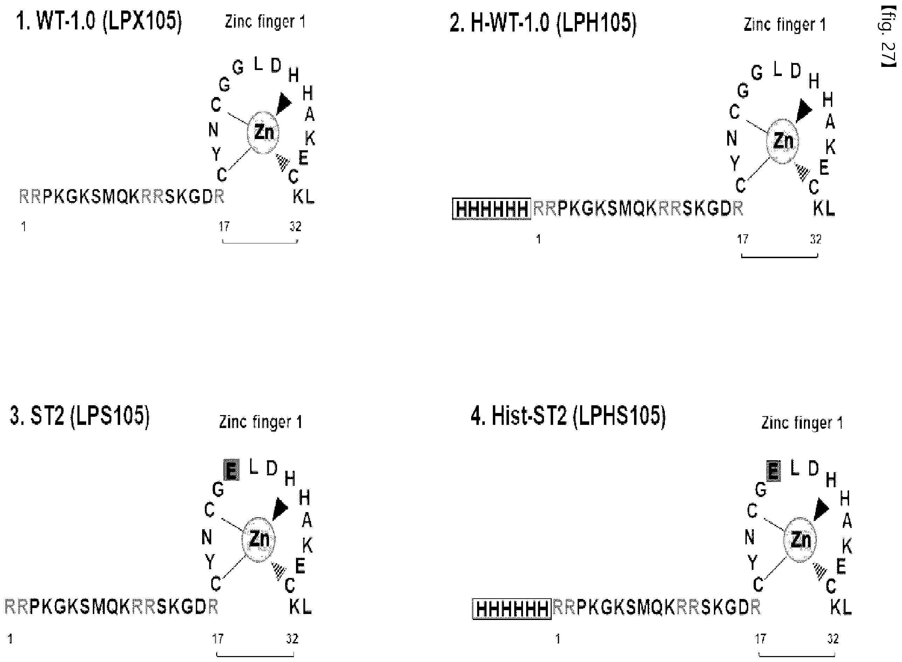

LPX105 variants were prepared using the method described in Example 3 above. Specifically, in order to confirm the effect of the zinc finger region mutation and Histidine tag insertion of LPX105 of SEQ ID NO: 37, LPS105 (SEQ ID NO: 38), LPH105 (SEQ ID NO: 39), and LPHS105 (SEQ ID NO: 40) were prepared by mutations in the zinc finger region of LPX105 and Histidine tag insertion (FIG. 27).

### 4-2. Confirmation of cell migration promotion of LPX105 variants

Using the method described in Example 1-2 above, the cell migration promotion of LPX105, LPH105, and LPHS105 was confirmed. Specifically, in order to confirm the cell migration effect, fibroblast 3T3 cells were treated with LPX105, LPH105, and LPHS105 peptides, incubated for 1 hour, and then washed. Thereafter, the migration of cells across a scratched boundary was measured 72 hours after treatment. The number of cells that migrated across a scratch line was measured by image analysis using ImageJ.

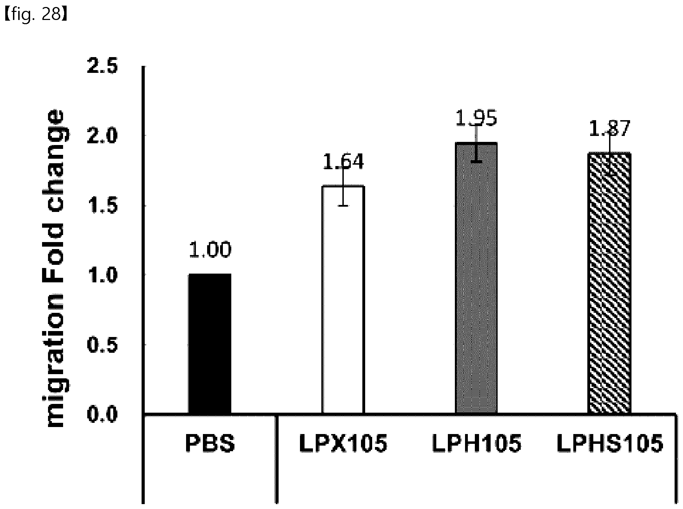

As a result, compared to a control group PBS, it was confirmed that LPX105 significantly promoted cell migration, and it was confirmed that LPH105 and LPHS105, variants of LPX105, increased cell migration more than LPX105 (FIG. 28).

### 4-3. Diabetic wound healing effect of LPX105 variants

By using the method described in Example 2-4 above, the diabetic wound healing effect of LPX105, LPH105, and LPHS105 was evaluated. Specifically, the skins of diabetes model mice (db/db) (n = 14 each) were punched with an 8 mm diameter puncher and treated with LPX105, LPH105 and LPHS105 (3 µg) or PBS daily for 10 days and subjected to wound dressing. The wound healing effect was confirmed by analyzing an initial wound area and a re-epithelialization area using ImageJ (mean SEM n = 3, *; p < 0.05).

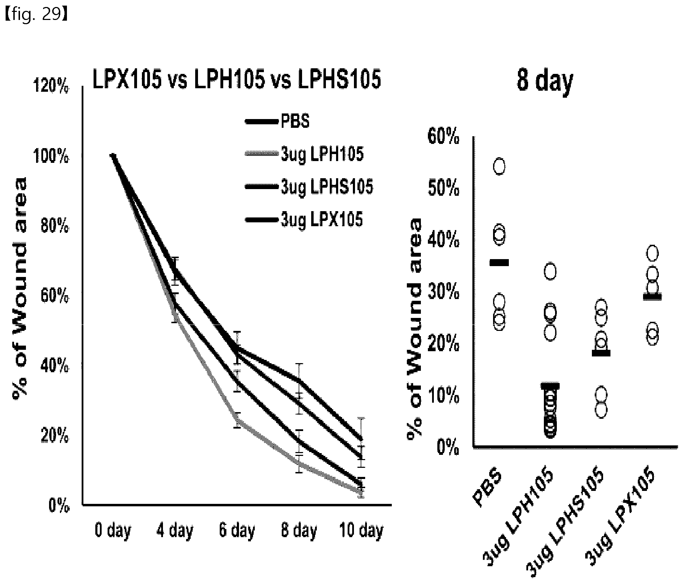

As a result, compared to the control group PBS, it was shown that LPX105 accelerated the diabetic wound healing in the db/db mouse model. In addition, it was confirmed that LPX105 variants LPH105 and LPHS105 accelerated the diabetic wound healing more significantly than LPX105 (FIG. 29).

## Claims

1. A cell-penetrating peptide comprising a cell-penetrating domain and a zinc finger domain.

2. The cell-penetrating peptide of claim 1, wherein the zinc finger domain is an amino acid sequence of Formula 1 below:
X1-X2-X3-X4-G-X5-L-D-X6-H-A-K-E-X7 (Formula 1, SEQ ID NO: 1)
Wherein,
X1 is C or A;
X2 is Y or R;
X3 is N, R or D;
X4 is C or A;
X5 is G or E;
X6 is H or A; and
X7 is C or A.

3. The cell-penetrating peptide of claim 2, wherein G-X8-R is added to an N-terminus of the zinc finger domain, wherein X8 is D or R.

4. The cell-penetrating peptide of claim 2, wherein K-X9 is added to a C-terminus of the zinc finger domain, wherein X9 is L or W.

5. The cell-penetrating peptide of claim 1, wherein the cell-penetrating domain is any one of amino acid sequences represented by SEQ ID NOs: 4 to 6.

6. The cell-penetrating peptide of claim 1, wherein the cell-penetrating peptide consists of an amino acid sequence represented by SEQ ID NO: 37.

7. The cell-penetrating peptide of claim 1, wherein the cell-penetrating peptide consists of an amino acid sequence represented by SEQ ID NO: 38.

8. The cell-penetrating peptide of claim 1, wherein the cell-penetrating peptide consists of an amino acid sequence represented by SEQ ID NO: 39.

9. The cell-penetrating peptide of claim 1, wherein the cell-penetrating peptide consists of an amino acid sequence represented by SEQ ID NO: 40.

10. The cell-penetrating peptide of claim 1, wherein the cell-penetrating peptide consists of any one sequence of amino acid sequences represented by SEQ ID NOs: 11 to 36.

11. The cell-penetrating peptide of claim 1, further comprising: amino acids E, GSE or His-tag at the N-terminus of the cell-penetrating domain.

12. The cell-penetrating peptide of claim 1, wherein a C-terminus of the zinc finger domain comprises any one of amino acid sequences represented by SEQ ID NOs: 7 to 10.

13. A polynucleotide encoding the peptide of claim 1.

14. A vector comprising the polynucleotide sequence of claim 13.

15. A host cell transformed with the vector of claim 14.

16. A pharmaceutical composition for wound healing comprising the peptide of claim 1 as an active ingredient.

17. The pharmaceutical composition for wound healing of claim 16, wherein the wound is a wound selected from the group consisting of non-healing traumatic wound, destruction of tissue by irradiation, abrasion, bone gangrene, laceration, avulsion, penetrated wound, gunshot wound, cuts, frostbite, contusion or bruise, skin ulcers, dry skin, skin keratosis, cracking, bursting, dermatitis, pain due to dermatophytosis, wounds such as surgical wound, vascular disease wound and corneal wound, bedsores, decubitus, conditions related to diabetes such as diabetic skin erosion and poor circulation, chronic ulcers, suture sites after plastic surgery, spinal injury wound, gynecological wound, chemical wound, and acne.

18. A pharmaceutical composition for preventing or treating angiogenesis-dependent diseases, comprising the peptide of claim 1 as an active ingredient.

19. The pharmaceutical composition for preventing or treating angiogenesis-dependent diseases of claim 18, wherein the angiogenesis-dependent disease is at least one selected from the group consisting of ischemic disease, wound, burns, psoriasis, chronic ulcer, myocardial infarction, angina, bedsores, hair loss, diabetic retinopathy, retinopathy of prematurity, age-related macular degeneration, glaucoma, diabetic ulcer, pulmonary hypertension and cerebrovascular dementia.

20. The pharmaceutical composition for preventing or treating angiogenesis-dependent diseases of claim 19, wherein the ischemic disease is at least one selected from the group consisting of cerebral ischemia, cardiac ischemia, diabetic vascular heart disease, heart failure, myocardial hypertrophy, retinal ischemia, ischemic colitis, ischemic acute renal failure, stroke, brain trauma, and neonatal hypoxia.

21. A method for wound healing comprising administering the cell-penetrating peptide of claim 1 to a wound in a pharmaceutically effective dose.

22. A method for treating angiogenesis-dependent diseases comprising administering the cell-penetrating peptide of claim 1 to a subject suffering from an angiogenesis-dependent disease in a pharmaceutically effective dose.

23. A use of a cell-penetrating peptide comprising a cell-penetrating domain and a zinc finger domain for use in the preparation of a composition for wound healing.

24. A use of a cell-penetrating peptide comprising a cell-penetrating domain and a zinc finger domain for use in the preparation of a composition for preventing or treating angiogenesis-dependent diseases.
